# EUROPEAN PATENT SPECIFICATION

(11) **EP 0 829 228 B1**
(45) Date of publication and mention of the grant of the patent: **27.10.2004**
(21) Application number: 97113868.0
(22) Date of filing: 11.08.1997
(51) Int. Cl.: A61B 5/044, G06T 1/00

(54) **Device for displaying currently connected measuring or therapeutic means in the body**
Vorrichtung zur graphischen Darstellung der im Körper gegenwärtigen angekoppelten Therapien- und messgeräte
Dispositif de représentation graphique d' appareillages de mesure ou thérapeutiques momentanément connectés au sein du corps

(30) Priority: 12.09.1996 SE 9603315
(43) Date of publication of application: 18.03.1998
(73) Proprietor: Siemens Aktiengesellschaft, 80333 München (DE)
(72) Inventor: Sjöholm, Gösta, 178 34 Ekerö (SE)

(56) References cited:
- US-A- 5 230 338
- US-A- 5 469 857

## Description

The present invention relates to medical device containing an input unit with a plurality of input terminals intended for the connection of measuring and/or therapeutic means, and a display device for displaying the currently connected measuring or therapeutic means.

A device for measuring electrical activity in the heart with inserted electrode catheters, equipped with a plurality of electrode poles, is known from EP, A1 0 614 678. Here, the means is devised to sense and show on the display the currently connected electrode poles and the electrode poles in contact with heart tissue.

In US 5,230,338 a system for tracking the movement of a single therapeutic (surgical instrument) means is disclosed, whereby with the aid of fiduical markers the location of the moved instrument is displayed overlaid on a image of the body region in which the instrument is being moved.

The object of the present invention is to make it possible to show on a display not only the currently connected measuring and/or therapeutic means, such as electrode poles, but even to generate a superimposed image of the organ or the environment inside the patient's body in which the measuring and/or therapeutic means is/are situated.

This objective is achieved with medical device of the initially described kind with the features set forth in claim 1.

The display device or monitor accordingly supplies an overview of the currently connected measuring and/or therapeutic means and its/their position in the organ or environment in which the measurement or therapy is performed. This greatly facilitates the work of the operator, usually a physician, performing the measurement or therapy.

According to an important embodiment of the unit according to the invention, the display device is arranged to display in the units measured by the measuring means. The display is thereby able to provide an overview of currently connected measuring and/or therapeutic means, the location of these means inside the patient's body and the magnitude of the units measured by the measuring means, a major advantage for the operator. The display device can advantageously be devised to display both text and graphic information. The aforesaid information can accordingly be displayed on the same monitor, in a highly functional fashion, preferably in the form of mixed graphic information and text information.

According to another advantageous embodiment of the device according to the invention, the measuring means are devised to measure ECG signals, blood pressure or oxygen pressure. Here, the equipment can also advantageously be devised to permit the monitoring of these parameters for a patient.

According to a further advantageous embodiment of the device according to the invention, the therapeutic means consists of a heart stimulator or ablation equipment. The heart stimulator can e.g. be of a type used for electrophysiological examinations and which delivers trains of pulses to the heart and ablation equipment of the kind used for electrophysiological therapy.

According to another advantageous embodiment of the device according to the invention, a switching device is arranged to establish connections, in an optional manner, between the equipment's input terminals and output terminals, thereby conveying great flexibility in the device's function and use.

According to a further advantageous embodiment of the device according to the invention, the display device has a touch screen which is arranged to show the equipment's input and output terminals, the desired connections between same being achieved when the screen is selectively touched. Connections between input and output terminals can therefore be achieved in a convenient manner.

According to another advantageous embodiment of the device according to the invention, the display device is arranged to display currently established connections, whereby the screen also supplies an overview of these currently established connections.

According to other advantageous embodiments of the device according to the invention, communications unit is arranged to communicate with a remote apparatus via a communications link in the form of e.g. an electric cable, an optical link or a telemetric link. The unit can be controlled from the said apparatus, e.g. computer equipment such as a PC, via the communications link and communications unit. The communiations unit can be devised to permit acquisition from the apparatus of measurement data over the input terminals, processing and analysis of acquired data and display of processed and acquired data on the display device. The display device can consist of a monitor in the apparatus or a separate display device.

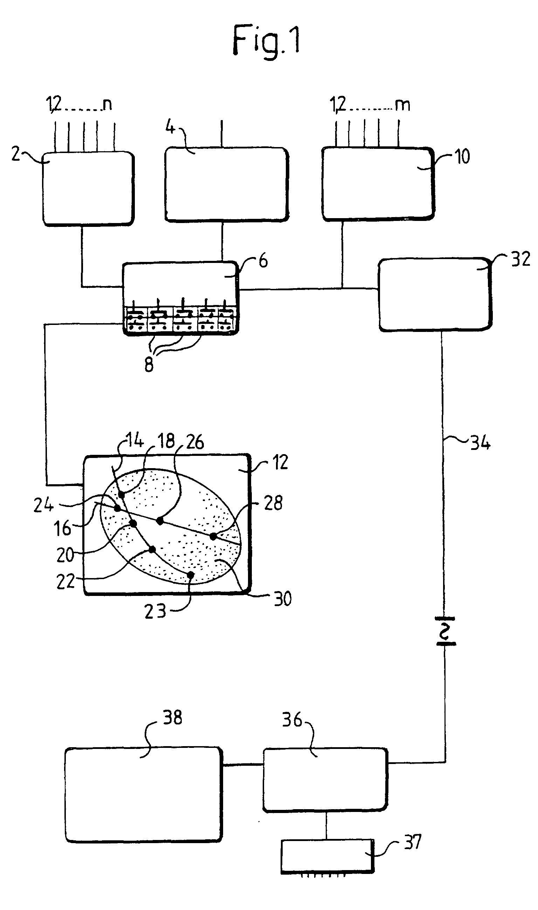

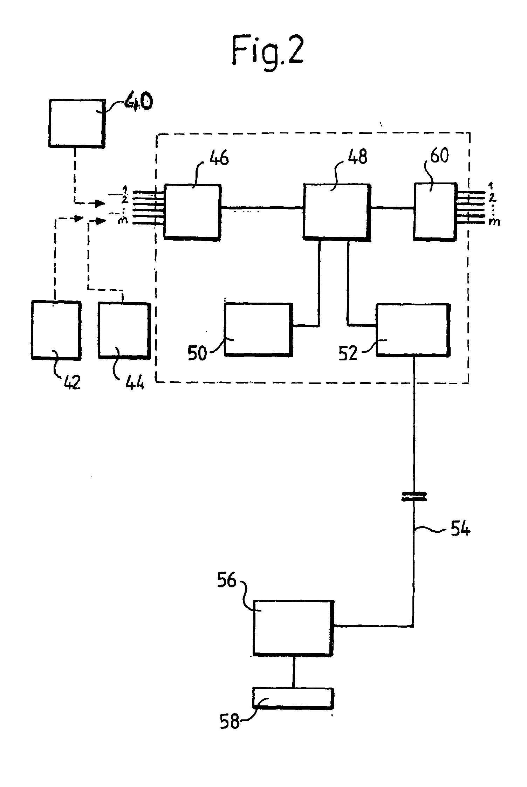

Selected exemplifying embodiments of the medical device according to the invention will now be described in greater detail, referring to attached drawings in which FIGS. 1 and 2 are block diagrams of two embodiments.

FIG. 1 shows a first embodiment of the medical device according to the invention with an input unit 2 with a plurality of input terminals 1,2,....n. The input unit 2 contains input electronics, such as an amplifier for amplifying input signals applied to these input terminals, and one or a plurality of processors and software for other appropriate signal processing.

The input unit 2 is connected to a switching device 6. The switching device 6 is appropriately devised as a switchbox with switching means in the form of activatable switches 8 to permit optional interconnection of the input unit's 2 input terminals 1,2...n and an output unit's 10 output terminals 1,2...m.

The input unit 2 can also be connected with the switching device 6 to a display device 12. Measuring and/or therapeutic means is/are intended for connection to the input terminals 1,2....n (see FIG. 2), and connection of the input terminals 1,2....n to the display device 12 makes it possible to display the currently connected measuring and/or therapeutic means.

Imaging means 4, such as X-ray machines, ultrasound equipment or equipment for imaging by mapping, are also connected for supplying image signals for transmission, via the switching device 6, to the display device 12, producing on same an image of the cavity or environment, e.g. an internal organ such as the heart, in which the one or more measuring and/or therapeutic means is/are situated, superimposed on the image of the currently connected measuring and/or therapeutic means. The display device 12 thereby supplies a direct overview of the currently connected measuring and/or therapeutic means and shows the position of these measuring and/or therapeutic means inside the internal organ.

The display device 12 shows two electrode catheters 14,16, inserted into the heart 30 of a patient. Each catheter 14,16 has a plurality of electrode poles 18,20,22,23 and 24,26,28 respectively. Of these, the electrode poles 18,23 designated with squares on the catheter 14 and 26,28 on the catheter 16 are connected to the medical device in question, whereas the other electrode poles 20,22 on the catheter 14 and 24 on the catheter 16 are not connected. As FIG. 1 shows, this electrode image is superimposed on an image of the heart 30 in which the electrode poles 18,20,22,23,24,26,28 are situated. So the display device 12 supplies an overview of the location in the heart 30 of both connected electrode poles 18,23,26,28 and disconnected electrode poles 20,22,24

The display device 12 can also advantageously be arranged to display signals or measurement values, determined by measuring means, in numeric or graphic form, the image supplied by the display device 12 thereby providing the operator with an even more detailed information overview.

The display device 12 can alternately be a touch screen arranged to display the input unit's input terminals and the output unit's output terminals, the desired connections between these terminals then being achieved when the operator selectively touches inter-terminal parts of the screen. This accordingly eliminates the need for a special switching device. Here, the display device is also arranged to display established connections.

Therapeutic means can consist of a heart stimulator of the kind which emits trains of pulses for use in e.g. electrophysiological examinations or ablation equipment used for electrophysiological treatment.

The measuring means can consist of ECG equipment, sphygmomanometers or device for measuring partial oxygen pressure. With such measuring means, medical device according to the invention can also be used in devices for patient monitoring.

A communications unit 32 is further arranged to communicate with remote equipment via a communications link 34 in the form of e.g. an electric cable, optical link or telemetry link. This equipment can contain a second switching device 36 and a second display device 38. The medical device can accordingly be operated from the said devices via the communications link 34 and the communications unit 32.

The second switching device 36 and the second display device 38 can advantageously be devised with computer equipment, such as a PC, the medical device then being operated with the computer's normal controls, such as a keyboard and mouse. Here, the function of the switching device is advantageously performed by the computer's control means. The display device can appropriately consist of the computer's monitor or of a separate display unit. In this manner, information can accordingly be displayed and device operated from one location at a distance from the input unit 2, an important advantage in certain applications such as monitoring.

In this version, the computer can be used for acquiring data from medical device and for processing and analyzing acquired data, in addition to serving as a display means.

The said devices can also incorporate a second output unit 37 with a plurality of output terminals 1,2....p which could send data to an additional device.

FIG. 2 shows a modified device in which information from the imaging means 40 for depicting the interior of a patient's body or an internal organ in her/his body and from measuring and therapeutic means 42,44, after processing and analysis in the input unit 46, is displayed, via a switching device 48, on a first display unit 50 or sent, via a communications unit 52 and a communications link 54, to a second display device 56 which also includes a control unit with associated control means 58 for controlling, in addition to the display of information, even the acquisition, processing and analysis of information in the input unit 46, as well as operation of the switching device, as described above in conjunction with the description of FIG. 1.

This embodiment also contains an output unit 60 through whose output terminals 1,2....m signals processed in the input unit 46 can be switched to other devices.

## Claims

1. A medical device containing an input unit (2,46) with a plurality of input terminals (1,2....n), to which measuring and/or therapeutic means (42,44), comprising electrode catheters (14,24) having located thereon a plurality of electrode poles (18,20,22,23,24,26,28), is/are intended for connection, and a display device (12,50) for displaying currently connected electrode poles (18,23,26,28) of the measuring and/or therapeutic means (42,44), **characterized in that** means (6;46,48) are provided to produce an image on the display device (12,50) of the cavity or the environment (30), in which the measuring and/or therapeutic means (42,44) is/are situated, onto which an image of the currently connected electrode poles (18,23,26,28) of the measuring and/or therapeutic means (42,44) and their respective positions inside the internal organ is/are superimposed.

2. Device according to claim 1, **characterized in that** the display device (12,50) is arranged to display the magnitude measured by the measuring means (42).

3. Device according to claim 1 or 2, **characterized in that** the said measuring means (42) are devised to measure ECG signals, blood pressure or oxygen pressure.

4. Device according to any of claims 1-3, **characterized in that** the said therapeutic means (44) contain a heart stimulator or ablation equipment.

5. Device according to any of claims 1-5, **characterized in that** the switching device (6,48) is arranged to establish connections, in an optional fashion, between the devices input terminals (1,2....n) and output terminals (1,2....m).

6. Device according to any of claims 1-4, **characterized in that** the display device (12) consists of a touch screen arranged to display the devices input (1,2....n) and output terminals (1,2....m), the desired connections between same being established when the operator selectively touches the screen.

7. Device according to claim 5 or 6, **characterized in that** the display device (12,50) is arranged to display established connections.

8. Device according to any of claims 1-7, **characterized in that** a communications unit (32,35) is arranged to communicate with a remote apparatus (36,56) via a communications link (34,54) in the form of e.g. an electric cable, optical link or telemetry link.

9. Device according to claim 8, **characterized in that** the device can be controlled from the said apparatus(36,56), via the communications link (34,54) and communications unit (32,52), in order to acquire measurement data across the input terminals (1,2....n), process and analyze acquired data in the input unit (2,46) and display processed and analyzed data on the display device (12,50).

10. Device according to claim 8 or 9, **characterized in that** the communications unit (32,52) is arranged to transmit information, intended for display on the display device (12,50), to a monitor in the said apparatus (38,56) or some separate display device.

11. Device according to claim 8, **characterized in that** the said apparatus contains a second switching device (36) and a second display device (38).

## Patentansprüche

1. Medizinische Einrichtung, die eine Eingabeeinheit (2, 46) mit mehreren Eingabeanschlüssen (1, 2,...n) enthält, an die Meß- und/oder Therapiemittel (42, 44), die Elektrodenkatheter (14, 24) mit mehreren darauf angeordneten Elektrodenpolen (18, 20, 22, 23, 24, 26, 28) umfassen, angeschlossen werden sollen, und eine Displayeinrichtung (12, 50) zum Anzeigen gegenwärtig angeschlossener Elektrodenpole (18, 23, 26, 28) der Meß- und/oder Therapiemittel (42, 44), **dadurch gekennzeichnet, daß** Mittel (6; 46, 48) vorgesehen sind zum Erzeugen eines Bilds auf der Displayeinrichtung (12, 50) des Hohlraums oder der Umgebung (30), in dem/der die Meß- und/oder Therapiemittel (42, 44) angeordnet sind, auf der ein Bild der gegenwärtig angeschlossenen Elektrodenpole (18, 23, 26, 28) der Meß- und/oder Therapiemittel (42, 44) und ihrer jeweiligen Positionen in dem inneren Organ überlagert ist.

2. Einrichtung nach Anspruch 1, **dadurch gekennzeichnet, daß** die Displayeinrichtung (12, 50) so angeordnet ist, daß sie die von den Meßmitteln (42) gemessene Größe anzeigt.

3. Einrichtung nach Anspruch 1 oder 2, **dadurch gekennzeichnet, daß** die Meßmittel (42) dafür ausgelegt sind, EKG-Signale, Blutdruck oder Sauerstoffdruck zu messen.

4. Einrichtung nach einem der Ansprüche 1-3, **dadurch gekennzeichnet, daß** die Therapiemittel (44) einen Herzstimulator oder ein Ablationsgerät enthalten.

5. Einrichtung nach einem der Ansprüche 1-5, **dadurch gekennzeichnet, daß** die Schalteinrichtung (6, 48) so angeordnet ist, daß sie wahlweise Verbindungen zwischen Eingabeanschlüssen (1, 2, ...n) und Ausgabeanschlüssen (1, 2,...m) der Einrichtungen herstellt.

6. Einrichtung nach einem der Ansprüche 1-4, **dadurch gekennzeichnet, daß** die Displayeinrichtung (12) aus einem Berührungsbildschirm besteht, der so angeordnet ist, daß er die Eingabeanschlüsse (1, 2,...n) und Ausgabeanschlüsse (1, 2,...m) der Einrichtungen anzeigt, wobei die gewünschten Verbindungen zwischen selbigen hergestellt werden, wenn der Bediener den Schirm selektiv berührt.

7. Einrichtung nach Anspruch 5 oder 6, **dadurch gekennzeichnet, daß** die Displayeinrichtung (12, 50) so angeordnet ist, daß sie hergestellte Verbindungen anzeigt.

8. Einrichtung nach einem der Ansprüche 1-7, **dadurch gekennzeichnet, daß** eine Kommunikationseinheit (32, 35) angeordnet ist, um mit einer abgesetzten Vorrichtung (36, 56) über eine Kommunikationsstrecke (34, 54) in Form zum Beispiel eines Stromkabels, einer optischen Strecke oder Telemetriestrecke zu kommunizieren.

9. Einrichtung nach Anspruch 8, **dadurch gekennzeichnet, daß** die Einrichtung von der Vorrichtung (36, 56) aus über die Kommunikationsstrecke (34, 54) und Kommunikationseinheit (32, 52) gesteuert werden kann, um Meßdaten über die Eingabeanschlüsse (1, 2,...n) zu erfassen, erfaßte Daten in der Eingabeeinheit (2, 46) zu verarbeiten und zu analysieren und verarbeitete und analysierte Daten auf der Displayeinrichtung (12, 50) anzuzeigen.

10. Einrichtung nach Anspruch 8 oder 9, **dadurch gekennzeichnet, daß** die Kommunikationseinheit (32, 52) so angeordnet ist, daß sie zur Anzeige auf der Displayeinrichtung (12, 50) gedachte Informationen an einen Monitor in der Vorrichtung (38, 56) oder eine beliebige separate Displayeinrichtung überträgt.

11. Einrichtung nach Anspruch 8, **dadurch gekennzeichnet, daß** die Vorrichtung eine zweite Schalteinrichtung (36) und eine zweite Displayeinrichtung (38) enthält.

## Revendications

1. Dispositif médical comprenant une unité (2,46) d'entrée ayant une pluralité de bornes (1, 2...n) d'entrées, auxquelles des moyens (42, 44) de mesure et /ou thérapeutiques, comprenant des cathéters (14, 24) à électrode sur lesquels sont placés une pluralité de pôles (18, 20, 22, 23, 24, 26, 28) à électrode, est ou sont destinés à se connecter, et un dispositif (12, 50) d'affichage pour afficher les pôles (18, 23, 26, 28) à électrode présentement connectés des moyens (42, 44) de mesure et/ou thérapeutiques, **caractérisé en ce qu'**il est prévu des moyens (6, 46, 48) de production d'une image sur le dispositif (12, 50) d'affichage de la cavité ou de l'environnement (30) dans lequel les moyens (42, 44) de mesure et/ou thérapeutiques est ou sont situés, sur laquelle est ou sont superposées une ou des images des pôles (18, 23, 26, 28) à électrode présentement connectés des moyens (42, 44) de mesure et/ou thérapeutiques et leur position respective à l'intérieur de l'organe interne.

2. Dispositif, suivant la revendication 1 ou 2, **caractérisé en** un dispositif (12, 50) d'affichage est conçu pour afficher la grandeur mesurée par les moyens (42) de mesure.

3. Dispositif, suivant la revendication 1 ou 2, **caractérisé en ce que** les moyens (42) de mesure sont conçus pour mesurer des signaux d'ECG, une pression sanguine ou une pression d'oxygène.

4. Dispositif suivant l'une quelconque des revendications 1 à 3, **caractérisé en ce que** les moyens (44) thérapeutiques comporte un stimulateur cardiaque ou un équipement d'ablation.

5. Dispositif suivant l'une quelconque des revendications 1 à 5, **caractérisé en ce que** dispositif (6, 48) de commutation est destiné à établir des connexions de la meilleure façon possible entre les bornes (1, 2 ... n) d'entrée des dispositifs et des bornes (1, 2 ... m) de sortie.

6. Dispositif suivant l'une quelconque des revendications 1 à 4, **caractérisé en ce que** le dispositif (12) d'affichage consiste en un écran tactile destiné à afficher les bornes (1, 2 ... n) d'entrée des dispositifs et les bornes (1, 2 ... m) de sortie, les connexions souhaitées entre celles-ci étant établies lorsque l'opérateur touche sélectivement l'écran.

7. Dispositif suivant la revendication 5 ou 6, **caractérisé en ce que** le dispositif (12, 50) d'affichage est destiné à afficher des connexions établies.

8. dispositif suivant l'une quelconque des revendications 1 à 7, **caractérisé en ce qu'**une unité (32, 35) de communication est destinée à communiquer avec un appareil (36, 56) éloigné par l'intermédiaire d'une liaison (34, 54) de communication sous la forme par exemple d'un câble électrique, d'une liaison optique ou d'une liaison par télémétrie.

9. Dispositif suivant la revendication 8, **caractérisé en ce que** le dispositif commandé par l'appareil (36, 56) par l'intermédiaire de la liaison (34, 54) de communication et de l'unité (32, 52) de communication afin d'acquérir des données de mesure aux les bornes (1, 2 ... n) d'entrée, de traiter et d'analyser des données acquises dans l'unité (2, 46) d'entrée et d'afficher des données traitées et analysées sur le dispositif (12, 50).

10. Dispositif suivant la revendication 8 ou 9, **caractérisé en ce que** l'unité (32, 52) de communication est destinée à transmettre de l'information destinée à être affichée sur les dispositifs (12, 50) d'affichage à un moniteur de l'appareil (38, 56) sur quelque dispositif distinct d'affichage.

11. Dispositif suivant la revendication 8, **caractérisé en ce** l'appareil comporte un deuxième dispositif (36) de commutation et un deuxième dispositif (38) d'affichage.
